(19) Europäisches Patentamt
European Patent Office
Office européen des brevets

(11) Publication number : **0 528 661 A2**

(12) # EUROPEAN PATENT APPLICATION

(21) Application number : **92307466.0**

(22) Date of filing : **14.08.92**

(51) Int. Cl.$^5$ : **C07C 271/22,** A61K 31/27, C07D 235/14

(30) Priority : **16.08.91 US 747153**

(43) Date of publication of application : **24.02.93 Bulletin 93/08**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Graham, Samuel L.**
**325 Widlund Drive**
**Schwenksville, PA 19473 (US)**
Inventor : **Ghosh, Arun K.**
**853 Jackson Street**
**Lansdale, PA 19947 (US)**
Inventor : **Huff, Joel R.**
**825 Surrey Drive**
**Gwynedd Valley, PA 19437 (US)**
Inventor : **Scholz, Thomas H.**
**127 South Front Street**
**Souderton, PA 18964 (US)**

(74) Representative : **Barrett-Major, Julie Diane et al**
**Merck & Co., Inc. European Patent Department Terlings Park Eastwick Road**
**Harlow Essex CM20 2QR (GB)**

(54) **HIV protease inhibitors with N-terminal polyether substituents.**

(57) Compounds of the form

A-G-B-B-J

wherein A is a polyether or ether substituent, G is a dipeptide isostere, B an amino acid or analog thereof, and J a small terminal group are described. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 528 661 A2

The present invention is concerned with compounds which inhibit the protease encoded by human immunodeficiency virus (HIV). The compounds, or pharmaceutically acceptable salts thereof, are of value in the prevention of infection by HIV, the treatment of infection by HIV and the treatment of the resulting acquired immune deficiency syndrome (AIDS).

The present invention also relates to pharmaceutical compositions containing the compounds and to a method of use of the present compounds and other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Inhibition of this processing prevents the production of normally infectious virus. For example, Kohl, N.E., et. al., Proc. Natl. Acad. Sci. USA, 85, 4686 (1988), demonstrated that genetic inactivation of the HIV encoded protease resulted in the production of immature, non-infectious virus particles. These results suggest that inhibition of the HIV protease represents a viable method for the treatment of AIDS and the prevention or treatment of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease.

The particular advantages of the compounds of the present invention are increased bioavailability and lowered toxicity. They are characterized as dipeptide isosteres with a polyether substituent only on the N-terminus.

## BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, hydrates or esters, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| | |
|---|---|
| | Activating Agent |
| HBT (HOBT or HOBt) | 1-hydroxybenzotriazole hydrate |
| | Condensing Agent |
| EDC | 1-ethyl-3-(3-dimethylamino-propyl)carbodiimide |
| | Deprotonating Agents |
| LDA | lithium diisopropylamide |
| | Other Reagents |
| TBDMSCl | t-butyl-dimethylsilylchloride |

DETAILED DESCRIPTION OF THE INVENTION AND PREFERRED EMBODIMENTS

This invention is concerned with the compounds of Formula I, combinations thereof, or pharmaceutically acceptable salts thereof, in the inhibition of HIV protease, the prevention of infection by HIV, the treatment of infection by HIV and in the treatment of the resulting acquired immune deficiency syndrome (AIDS). Compounds of formula I are defined as follows:

A-G-B-B-J I,

wherein A is

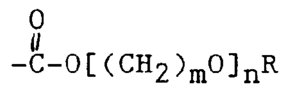

where n = 1-6, m may be 1-3 within each repeating unit n, and R is either hydrogen or $C_{1-4}$ alkyl.

G is

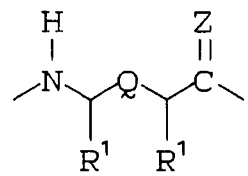

wherein Z is O, S, or NH, and

$R^1$ is independently

1) hydrogen

2)

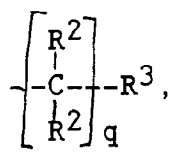

3) -OR, wherein R is H, or $C_{1-4}$ alkyl,

4) $-NR_2$,

5) $C_{1-4}$ alkylene-$R^3$;

wherein q is 0-5 and $R^2$ is independently

a) hydrogen,

b) hydroxy, or

c) $C_{1-4}$-alkyl;

$R^3$ is

a) hydrogen,

b) aryl, unsubstituted or substituted with one or more of

i) halo,

ii) hydroxy,

iii) $-NH_2$, $-NO_2$, -NHR, or $-NR_2$, wherein R is H, or $C_{1-4}$ alkyl,

iv) $C_{1-4}$ alkyl,

v) $C_{1-3}$ alkoxy,

vi) -COOR,

vii)

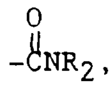

viii) $-CH_2NR_2$,
ix)

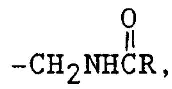

x) CN,
xi) $CF_3$,
xii)

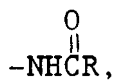

xiii) aryl $C_{1-3}$ alkoxy,
xiv) aryl,
xv) $-NRSO_2R$,
xvi) $-OP(O)(OR_x)_2$ wherein $R_x$ is H or aryl, benzyl or a metal ion,
xvii)

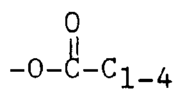

alkyl substituted with one or more of amine or quaternary amine;

$R^3$ may also be

c) 5 or 6 membered heterocycle including up to 3 heteroatoms selected from N, O, and S, such as imidazolyl, thiazolyl, furyl, oxazolyl, piperidyl, thiadiazolyl, piperazinyl, pyridyl, or pyrazinyl, any of which heterocycle may be unsubstituted or substituted with one or more of

i) halo,
ii) hydroxy,
iii) $-NH_2$, -NHR, $-NR_2$,
iv) $C_{1-4}$ alkyl,
v) $C_{1-3}$ alkoxy,
vi) -COOR,
vii)

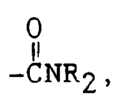

viii) $-CH_2NR_2$,
ix)

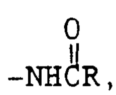

x) -CN,
xi) $CF_3$,
xii) $-NHSO_2R$,

xiii) -OP(O)($OR_x$)2 wherein $R_x$ is H or aryl, or
xiv)

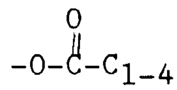

alkyl-substituted with one or more of amine or quaternary amine;
xv) SR, S(O)R and $S(O_2)R$;

d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)

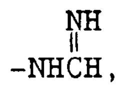

v)

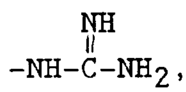

vi) -COOH,
vii)

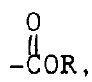

viii) -SR,S(O)R and $S(O)_2R$,
ix) $-SO_2NHR$,
x) $C_{1-4}$ alkyl sulfonyl amino or aryl sulfonyl amino,
xi) -CONHR,
xii)

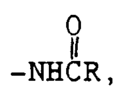

xiii) -OR,
xiv) aryl $C_{1-3}$ alkoxy or,
xv) aryl;

e) $C_{3-7}$ cycloalkyl unsubstituted or substituted with one or more of
i) hydroxy,
ii) $C_{1-4}$ alkyl,
iii) $-NH_2$, -NHR, $-NHR_2$,
iv)

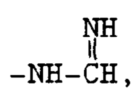

v)

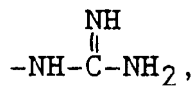

vi) -COOH,
vii)

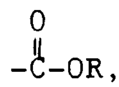

viii) -SR,S(O)R and $S(O_2)R$
ix) $-SO_2NH_2$,
x) alkyl sulfonylamino or aryl sulfonylamino,
xi) -CONHR, or
xii)

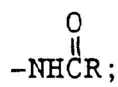

f) a 5- to 7-membered carbocyclic or 7- to 10-membered bicyclic carbocyclic ring which is either saturated or unsaturated, such as cyclopentane, cyclohexane, indan, norbornane, or naphthalene, the carbocyclic ring being unsubstituted or substituted with one or more of
i) halo
ii) -OR, wherein R is H or $C_{1-4}$ alkyl,
iii)

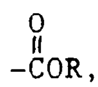

iv)

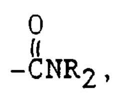

v) $-CH_2NR_2$,
vi) $-SO_2NR_2$ or $-S(O)_y$ R wherein y is 0,1 or 2,
vii) $-NR_2$,
viii)

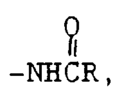

ix) $C_{1-4}$ alkyl,
x) phenyl,
xi) $-CF_3$, or R
xii) $-N-SO_2R$;
$R^9$ is -OH or $-NHR^{10}$, wheren $R^{10}$ is -H,

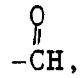

$-C_{1-4}-$ alkyl or -COOR; and

Q is

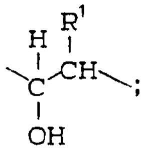; 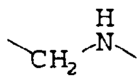; 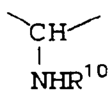; 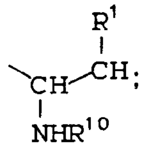; 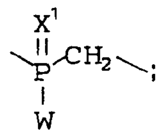; or 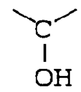,

wherein $R^1$ and $R^{10}$ are defined above; $X^1$ is O, S, or NH; and

W is

1) OH,

2) $NH_2$

3) OR, or

4) NHR;

B is, independently, absent, or

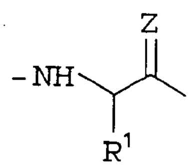,

J is

1) $YR^{11}$ wherein:

Y is O or NH, and

$R^{11}$ is

a) H;

b) $C_{1-6}$ alkyl, unsubstituted or substituted with one or more of

i) $-NR^6_2$,

ii) -OR,

iii) $-NHSO_2C_{1-4}$ alkyl,

iv) $-NHSO_2$ aryl, or $-NHSO_2$ (dialkylaminoaryl),

v) $-CH_2OR$,

vi) $C_{1-4}$ alkyl,

vii)

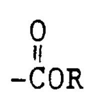,

viii)

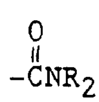,

ix)

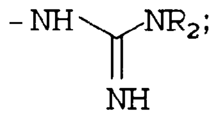

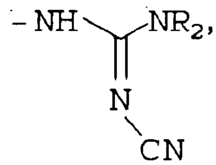

x)

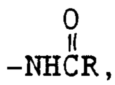

xi)

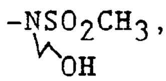

xii)

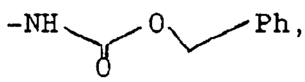

xiii) $-NR_3^{\oplus}A^{\ominus}$ wherein $A^{\ominus}$ is a counterion,

xiv) $-NR^{12}R^{13}$ wherein $R^{12}$ and $R^{13}$ are the same or different and are $C_{1-5}$ alkyl joined together directly to form a 5-7 membered heterocycle,

xv) aryl,

xvi) -CHO,

xvii) $-OP(O)(OR_x)_2$ wherein $R_x$ is H or aryl, or

xviii)

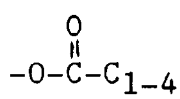

alkyl substituted with one or more of amine or quaternary amine;

c) $-(CH_2CH_2O)_nCH_3$ or $-(CH_2CH_2O)_nH$;

2) $-N(R^{11})_2$,

3) $-NR^{12}R^{13}$ wherein $R^{12}$ and $R^{13}$ are defined above, or

4)

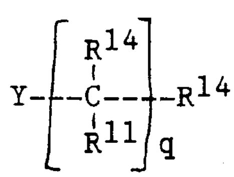

wherein:

Y, $R^{11}$, and q are defined above, and

$R^{14}$ is

a) hydrogen;

b) aryl unsubstituted or substituted with one or more of

i) halo,

ii) -OR, wherein R is H or $C_{1-4}$ alkyl,

iii)

$$-\overset{O}{\overset{\|}{C}}OR,$$

iv)

$$-\overset{O}{\overset{\|}{C}}NR_2,$$

v) $-CH_2NR_2$,

vi) $-SO_2NR_2$,

vii) $-NR^2$,

viii)

$$-NH\overset{O}{\overset{\|}{C}}R,$$

ix) $C_{1-4}$ alkyl,

x) phenyl,

xi) $-CF_3$,

xii)

$$-\overset{R}{\overset{|}{N}}-SO_2R,$$

xiii) $-C_{1-4}$ alkyl-$NR_2$,

xiv) $-OP(O)(OR_x)_2$ wherein $R_x$ is H or aryl, or

xv)

$$-O-\overset{O}{\overset{\|}{C}}-C_{1-4}$$

alkyl substituted with one or more of amine or quaternary amine;

c) heterocycle, unsubstituted or substituted with one or more of

i) halo,

ii) -OR, wherein R is H, $C_{1-4}$ alkyl, or $C_{1-4}$ alkenyl,

iii)

$$-\overset{O}{\overset{\|}{C}}OR,$$

iv)

$$-\overset{O}{\overset{\|}{C}}NR_2,$$

v) $-CH_2NR_2$,
vi) $-SO_2NR_2$,
vii) $-NR_2$,
viii)

$$-NH\overset{O}{\overset{\|}{C}}R,$$

ix) $C_{1-4}$ alkyl,
x) phenyl,
xi) $-CF_3$,
xii)

$$-\overset{R}{\overset{|}{N}}-SO_2R,$$

xiii) phenyl $C_{1-4}$ alkyl,
xiv)

$$-O\overset{O}{\overset{\|}{C}}R,$$

xv) $-OP(O)(OR_x)_2$ wherein $R_x$ is H or aryl, or
xvi)

$$-O-\overset{O}{\overset{\|}{C}}-C_{1-4}$$

alkyl substituted with one or more of amine or quaternary amine;

d) A 5- to 7-membered carbocyclic or 7- to 10-membered bicyclic carbocyclic ring which is either saturated or unsaturated, such as cyclopentane, cyclohexane, indan, norbornane or naphthalene, the carbocyclic ring being unsubstituted or substituted with one or more of

i) halo,
ii) -OR, wherein R is H or $C_{1-4}$ alkyl,
iii)

$$-\overset{O}{\overset{\|}{C}}OR,$$

iv)

$$-\overset{O}{\overset{\|}{C}}NR_2,$$

v) $-CH_2NR_2$,
vi) $-SO_2NR_2$,
vii) $-NR_2$,

viii)

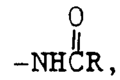

ix) $C_{1-4}$ alkyl,
x) phenyl,
xi) $-CF_3$,
xii)

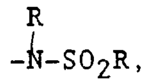

xiii) $-OP(O)(OR_x)_2$ wherein $R_x$ is H or aryl, or
xiv)

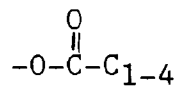

alkyl substituted with one or more of amine or quaternary amine;

or pharmaceutically acceptable salts, hydrates or esters thereof.

In the compounds of the present invention, the A, G, B and J components and the like may have asymmetric centers and occur as racemates, racemic mixtures and as individual diastereomers or enantiomers, with all isomeric forms being included in the present invention.

When any variable (e.g., aryl, heterocycle, R, $R^9$, $R^{10}$, $R^{11}$, $R^{12}$, n, Z, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge. "Alkenyl" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle or heterocyclic, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzocthiopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The pharmaceutically-acceptable salts of the compounds of Formula I (in the form of water- or oil-soluble

or dispersible products) include the conventional non-toxic salts or the quaternary ammonium salts of these compounds, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Esters are also encompassed by the present invention. and include those which would readily occur to the skilled artisan, for example, $C_{1-4}$ alkyl esters.

In a preferred embodiment of this invention,

A is

$$-\overset{O}{\overset{\|}{C}}O[(CH_2)_2O]_nCH_3,\ n=1-4;$$

and

G is

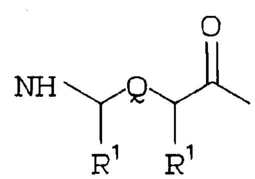

A second embodiment is further limited to compounds wherein

G is

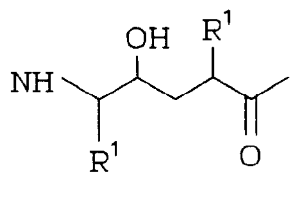

;

and B is absent or present once.

A third embodiment is further limited to compounds wherein B is absent, J is $NHR^{14}$ and $R^{14}$ is a substituted 5- to 7-membered carbocyclic or heterocyclic ring or a substituted 7- to 10-membered bicyclic carbocycle or heterocycle which may be saturated or unsaturated.

A fourth embodiment is further limited to compounds wherein J is indan, substituted once or twice with OH.

Novel compounds of the invention include, but are not limited to:

N-(2(R)-hydroxy-1(S)-indanyl)-5(S)-(3,6,9,12-tetraoxatridecyloxycarbonylamino)-4(S)-hydroxy-2(R)-phenylmethyl-6-phenylhexanamide,

N-(2(R)-hydroxy-1(S)-indanyl)-5(S)-(3,6,9,12-tetraoxatridecyloxycarbonylamino)-4(S)-hydroxy-2(R)-(3-phenylprop-2-en-1-yl)-6-phenylhexanamide,
N-(2(R)-hydroxy-1(S)-indanyl)-5(S)-(3,6,9-trioxadecyloxycarbonylamino)-4(S)-hydroxy-2(R)-phenylmethyl-6-phenylhexanamide,
N-(2(R)-hydroxy-1(S)-indanyl)-5(S)-(3,6-dioxaheptyloxycarbonylamino)-4(S)-hydroxy-2(R)-phenylmethyl-6-phenylhexanamide,
N-(2(R)-hydroxy-1(S)-indanyl)-5(S)-(3-oxabutyloxycarbonylamino)-4(S)-hydroxy-2(R)-phenylmethyl-6-phenylhexanamide,
N-(2(R)-hydroxy-1(S)-indanyl)-5(S)-(3,6,9-trioxadecyloxycarbonylamino)-4(S)-hydroxy-2(R)-(3-phenylprop-2-en-1-yl)-6-phenylhexanamide,
N-(2(R)-hydroxy-1(S)-indanyl)-5(S)-(3,6-dioxaheptyloxycarbonylamino)-4(S)-hydroxy-2(R)-(3-phenylprop-2-en-1-yl)-6-phenylhexanamide,
N-(2(R)-hydroxy-1(S)-indanyl)-5(S)-(3-oxabutyloxycarbonylamino)-4(S)-hydroxy-2(R)-(3-phenylprop-2-en-1-yl)-6-phenylhexanamide,
N-(2-benzimidazolylmethyl)-5(S)-(3,6,9-trioxadecyloxycarbonylamino)-4(S)-hydroxy-6-phenyl-2(R)-(3-phenylprop-2-en-1-yl)hexanoyl isoleucylamide,
N-[(cis)-2(R)-hydroxy-1(S)-indanyl)-5(S)-[(2-hydroxyethoxycarbonyl)amino]-4(S)-hydroxy-6-phenyl-2(R)-(phenylmethyl)]hexanamide,
N'-[2-hydroxyethoxy)carbonyl-5(S)-amino-4(S)-hydroxy-6-phenyl-2(R)-(3-phenylprop-2-en-1-yl)hexanoyl]-N-isoleucine-(2-benzimidazolylmethyl)amide,
N-[(cis)-2(R)-hydroxy-1(S)-indanyl]-5(S)-[(2-hydroxyethoxycarbonyl)amino)-4(S)-hydroxy-6-phenyl-2(R)-(3-phenylprop-2-en-1-yl)hexanamide,
N-(2(R)-hydroxy-1(S)-indanyl)-5(S)-(3,6,9,12-tetraoxatridecyloxycarbonylamino)-4(S)-hydroxy-6-phenyl-2(R)-(4-hydroxyphenyl)methylhexanamide,
N-(2(R)-hydroxy-1(S)-indanyl)-5(S)-[(3-hydroxy)propoxycarbonyl]amino-4(S)-hydroxy-6-phenyl-2(R)-[3-phenylpropanyl]hexanamide,
N-(2(R)-hydroxy-1(S)-indanyl)-5(S)-[(3-methoxy)propoxycarbonyl]amino-4(S)-hydroxy-6-phenyl-2(R)-[3-phenylprop-2-en-1-yl]hexanamide, or
N-(2(R)-hydroxy-1(S)-indanyl)-5(S)-(13,13-dimethyl-3,6,9,12-tetraoxatetradecyloxycarbonylamino)-4(S)-hydroxy-2(R)-phenylmethyl-6-phenylhexanamide, or pharmaceutically acceptable salt or ester therof.

The most preferred compound of this invention is:

<u>Compound A:</u>

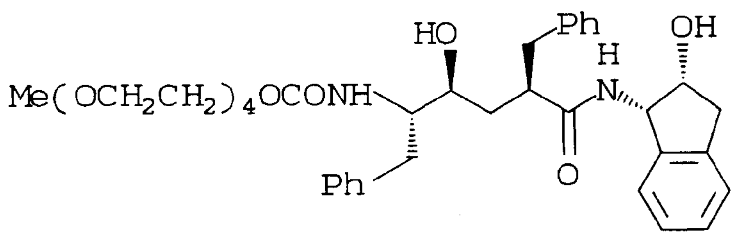

N-(2(R)-hydroxy-1(S)-indanyl)-5(S)-(3,6,9,12-tetraoxatridecyloxycarbonylamino)-4(S)-hydroxy-2(R)-phenylmethyl-6-phenylhexanamide;

The compounds of the present invention are prepared in accordance with Schemes I-III. In general, once the G substituent is made, the rest of the synthesis follows the principle of amide bond formation by the coupling methods of either solution-phase or solid-phase peptide synthesis. The addition and removal of one or more protecting groups is also typical practice.

Amide couplings for synthesizing compounds of this invention are typically performed by the carbodiimide method with reagents such as dicyclohexylcarbodiimide, or 1-ethyl-3-(3-dimethylaminopropyl) carbodiimide. Other methods of forming the amide or peptide bond include, but are not limited to synthetic routes via an acid chloride, azide, mixed anhydride or activated ester. Typically, solution phase amide couplings are performed, but solid-phase synthesis by classical Merrifield techniques may be employed instead.

Some schemes for preparing compounds of formula I are presented below. The examples specifically illustrate the application of the following schemes to specific compounds. Additional related information on synthetic background is contained in EPO 0337714.

One scheme for the preparation of the key intermediates involved in this invention is as follows:

SCHEME I

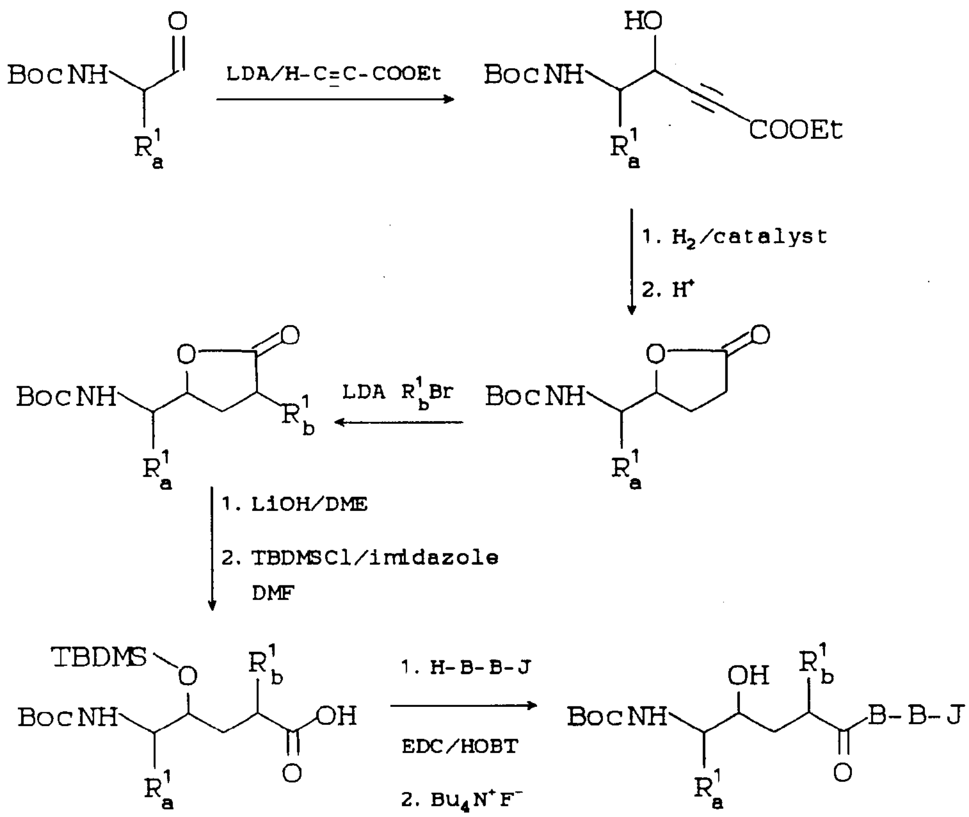


wherein $R^1_a$ and $R^1_b$ are each independently $R^1$.

Carbamates which comprise the current invention are prepared according to the following Scheme:

## SCHEME II

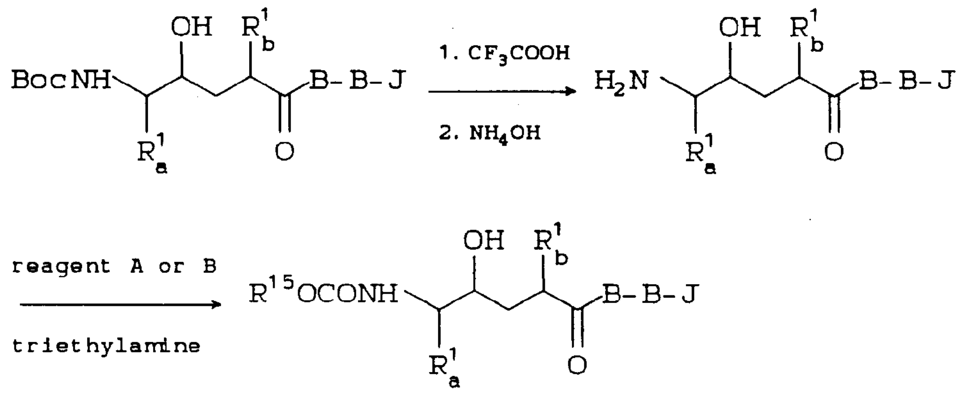


Reagents A and B for generating the carbamates are prepared by the reaction of the appropriate alcohol with p-nitrophenyl chloroformate or sequentially with phosgene (or triphosgene) and N-hydroxy succinimide according to the reactions:

1)

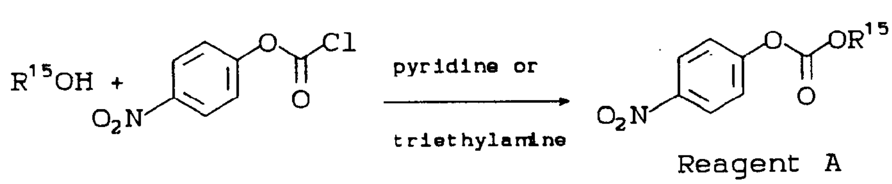


Reagent A

2)

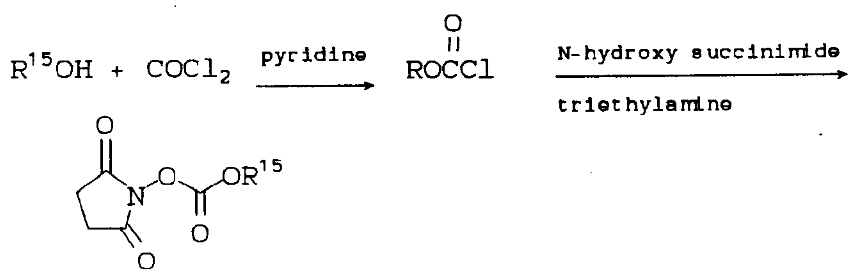


Reagent B

wherein $R^{15}$ is $HO[(CH2)_mO]_n$ $R^{17}$ and $R^{17}$ is hydrogen, $C_{1-4}$ alkyl or an alcohol protecting group such as benzyl, trialkysilyl.

Alternatively, several of the key intermediates in the synthesis of the compounds disclosed in this invention are prepared by the following reaction:

SCHEME III

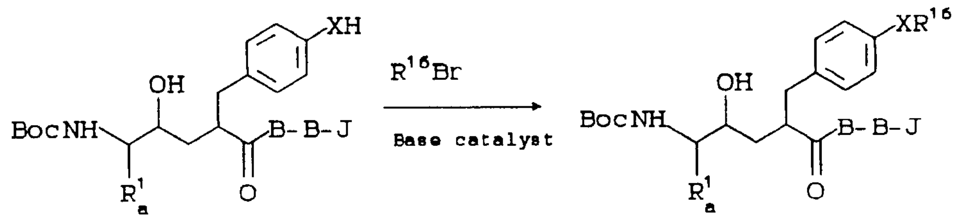


wherein $R^{16}Br$ is representative of any alkylating agent, as within the definition of $R^1$.

Additional schemes and methods of synthesis are found in EPO 0,337,714. Other relevant publications are U.S. Patent 4,661,473; Evans, B.E. et al., J. Org. Chem. 50, 4615 (1985); Luly, J.R. et al., ibid., 52, 1487 (1987).

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymtomatic, and actual or potential exposure to HIV. For example, the compounds of this invention are useful in treating infection by HIV after suspected past exposure to HIV by e.g., blood transfusion, accidental needle stick, or exposure to patient blood during surgery.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono= or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age of the patient, body weight, general health, sex, diet, mode and time of administration, rate of excretion, drug combination, the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the following Table [source: Marketletter, Nov. 30. 1987, pp. 26-27; Genetic Engineering News, Jan. 1988, Vol. 8, 23]:

TABLE[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| ddI (dideoxyinosine) | Bristol-Myers | AIDS, ARC |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological involvement. |

| Drug Name | Manufacturer | Indication |
| --- | --- | --- |
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with AZT |
| ZOVIRAX (acyclovir) | Burroughs Wellcome | AIDS, ARC, in comb with AZT |

B. Immunomodulators

| Drug Name | Manufacturer | Indication |
| --- | --- | --- |
| ABPP KS (bropirimine) | Upjohn | Advanced AIDS, |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |

| Drug Name | Manufacturer | Indication |
|---|---|---|
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & AZT therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

EXAMPLE 1

N-(2(R)-hydroxy-1(S)-indanyl)-5(S)-(3,6,9,12-tetraoxatridecyloxycarbonylamino)-4(S)-hydroxy-2(R)-phenylmethyl-6-phenylhexanamide

Step A: N-(1,3,6,9,12,15-hexoxa-2-oxohexadecyl)succinimide

Tetraethylene glycol monomethyl ether (4.16 g) was added to a stirred solution of phosgene (12.5%) in toluene (80 ml). After four hours excess phosgene was blown out of the reaction mixture into a sodiumhydroxide aqueous ethanol scrubbing solution using argon gas. The reaction mixture was concentrated in vacuo. The resulting oil residue was dissolved in acetonitrile (50 ml) and to this solution was added N-hydroxy succinimide (2.85 g) and triethyl amine (3.4 ml). After stirring for 30 minutes the reaction mixture was filtered to remove a white solid and the filtrate was concentrated in vacuo. The resulting semi-solid residue was partitioned between ethyl acetate and 10% citric acid solution. The organic phase was separated, washed with water then brine, dried ($MgSO_4$), filtered and concentrated in vacuo to 5.67 g of the title compound as an oil.
$^1$H NMR ($CDCl_3$): $\delta$ 2.84 (s, 4H); 3.38 (s, 3H); 3.55 (m, 2H); 3.65 (m, 10H); 3.80 (t, 2H); 4.48 (t, 2H).

Step B: Preparation of 3(S)-[(1,1-Dimethylethoxycarbonyl)amino]-2(RS)-hydroxy-4-phenyl-1-trimethylsilylbutane

To a stirred suspension of magnesium turnings (9.79 g, 403 mmol) in dry diethyl ether (200 ml) under nitrogen was added chloromethyltrimethylsilane (50 ml, 358 mmol). The reaction was initiated by gentle warming and then was cooled in an ice bath to maintain gentle reflux. After the exotherm was complete the reaction was stirred at room temperature for 1 hour then cooled to 78°C in a dry ice/acetone bath. To the solution of the Grignard was added dropwise with stirring a solution of N-2(S)-[(1,1-dimethylethoxycarbonyl)amino]-3-phenyl propionaldehyde (19.3 g, 77.4 mmol) in dry diethyl ether (250 ml) dropwise such that the temperature of the reaction remained below -55°C. The resultant gray suspension was allowed to warm to room temperature where it was stirred for 30 minutes then was quenched by pouring into a mixture of ice (500 g) and 10% citric acid (500 ml). The organic phase was collected and the aqueous phase was extracted with diethyl ether (3X300 ml). The combined organics were washed with 10% citric acid (1X300 ml) and brine (1X200 ml), dried over anhydrous magnesium sulfate, filtered, and concentrated to give crude 3(S)-[(1,1-dimethylethoxycarbonyl)amino]-2(RS)-hydroxy-4-phenyl-1-trimethylsilyl butane (26.6 g, quantitative crude yield) as a yellow oil, an analytical sample was obtained by low pressure chromatography (silica gel, 230-400 mesh; diethyl ether:hexanes, 30%:70%) followed by recrystallization from heptane. mp = 91-95°C;

elemental analysis, calcd. for $C_{18}H_{31}NO_3Si$ (337.53):
C, 64.05; H, 9.26; N, 4.15;
Found: C, 64.15; H, 9.13; N, 4.22; $[\alpha]_D^{20} = -40.0°$

Step C: Preparation of 3(S)-Amino-4-phenyl-1-butene

To a stirred solution of the product of Step B (22.8 g, 67.5 mmol) in dry methylene chloride (400 ml) cooled in an ice bath and under nitrogen was added in a fine stream boron trifluoride etherate (43 ml, 345 mmol). The solution was allowed to warm to room temperature where it was stirred for 4 days. Reaction was cooled in an ice bath and quenched by the dropwise addition to 10% sodium hydroxide (400 ml). The organic phase was collected and the aqueous phase was extracted with methylene chloride (2X250 ml). The combined organics were washed with brine (1X200 ml), dried over anhydrous magnesium sulfate, filtered, and concentrated to give crude 3(S)-amino-4-phenyl-1-butene (14.2 g) as a yellow oil.

Step D: Preparation of N-3(S)-[(1,1-Dimethylethoxycarbonyl)amino]-4-phenyl-1-butene

A solution of the product of Step C (14.2 g) and di-tert-butyl dicarbonate (31.0g, 142 mmol) in dry methylene chloride (200 ml) was stirred at room temperature for 18 hours, washed with 10% citric acid (3X100 ml), water (1X100 ml), sat'd. sodium bicarbonate (3X125 ml), and brine (1X250 ml), dried over anhydrous magnesium sulfate, filtered, and concentrated to yield crude N-3(S)-[(1,1-dimethylethoxycarbonyl)amino]-4-phenylbutene (34.6 g) as a yellow oil. Crude product was purified by low pressure chromatography (silica gel, 230-400 mesh, 10x20 cm column; diethyl ether:hexanes, 20%:80%) to yield N-3(S)-[(1,1-dimethylethoxycarbonyl)amino]-4-phenyl-1-butene (16.3 g, 97.6% yield) as a white solid. An analytical sample was obtained by recrystallization from heptane. mp = 67.5-68.5°C;

elemental analysis, calcd. for $C_{15}H_{21}NO_2$ (247.34):
C, 72.84; H, 8.56; N, 5.66;
Found: C, 72.78; H, 8.76; N, 5.64.

Step E: Preparation of 1(R)-[1′(S)-(1,1-Dimethylethoxycarbonyl)amino-2-phenylethyl]oxirane

To a solution of the product of Step D (9.4 g, 38 mmol) in dry methylene chloride (100 ml) cooled in an ice bath and under nitrogen was added 3-chloroperoxybenzoic acid (technical grade, 80-85%; 41 g, -200 mmol). The mixture was stirred at 0°C for 18 hours and at 25°C for 23 hours., then diluted with diethyl ether (300 ml), and poured in ice cold aq. 10% sodium sulfite (1 L). The organic layer was collected and the aqueous layer was extracted with diethyl ether (3X100 ml). The combined organics were washed with 10% sodium sulfate (3 x 100 ml), sat'd. sodium bicarbonate (3X100 ml), and brine (1x100 ml), dried over anhyd. sodium sulfate, filtered and concentrated to give a white solid. Crude product was purified by low pressure chromatography (silica gel 230-400 mesh, 8 X 15 cm column; ethyl acetate:hexanes, 25%:75%) to yield 1(R)-[1′(S)-(1,1-dimethylethoxycarbonyl)amino-2-phenylethyl]oxirane (7.0 g, 70% yield) as a clear oil which crystallized upon standing. An analytical sample was obtained by recrystallization from heptane. mp = 51.5-52°C; elemental analysis, calcd. for $C_{15}H_{21}NO_3$ (263.34):

C, 68.42; H, 8.04; N, 5.32;
Found: C, 68.22; H, 8.26; N, 5.29; $[\alpha]_D^{20} = -1.34°$.

Step F: Preparation of (5S, 1′S)-3-carboethoxy-5-(1-(1,1-dimethylethoxycarbonyl)amino)-2-phenylethyl)-dihydrofuran-2-(3H)-one

The product from Step E, 9.93 g, was dissolved in 100 mL of absolute ethanol and added to a solution of 2.6g of sodium and 20.1 mL of diethyl malonate in 170 mL of absolute ethanol. After stirring overnight, the reaction was acidified to pH~4 with 10% citric acid and extracted with 2X 500 mL of ether. The combined organic extracts were washed 1X500 mL $H_2O$, 1X500 mL sat'd $NaHCO_3$, 1X500 mL sat'd brine and dried over $MgSO_4$. The solvents were removed and the crude product purified by low pressure chromatography on silica gel eluting with 50% ether/hexanes (or EtOAc/hexane). The yield of semi-solid product was 10.6g. The later fractions contained 2.5 g of the undesired 5R isomer as a white solid.

Step G: Preparation of (5S, 1′S)-3-carboethoxy-3-phenylmethyl-5-(1-((1,1-dimethylethoxycarbonyl)amino)-2-phenylethyl)dihydrofuran-2-(3H)-one

The product of Step F, 10.6 g, was dissolved in 100 mL of abs. ethanol containing 3.7 mL of benzyl bromide and added to a solution of 0.71 g of sodium in 100 mL of absolute ethanol. The solution was heated to 50°C for 1.5 hours, then cooled in an ice bath and acidified with 500 mL of 10% citric acid. The mixture was extracted 3X500 mL of ether and the combined ether extracts washed with 400 mL of $H_2O$, 400 mL of brine, dried ($MgSO_4$) and the solvent removed under reduced pressure to give 13.6 g of a clear colorless oil which was essentially homogeneous by TLC (25% ethyl acetate/hexanes).

Step H: Preparation of (3R, 5S, 1′S)-3-Benzyl-5-(1-((1,1-dimethylethoxycarbonyl)amino)-2-phenylethyl)dihydrofuran-2-(3H)-one

The product of Step G, 13.6 g, was dissolved in 250 mL of 1,2-dimethoxy ethane, and to it was added 117 mL of 1M lithium hydroxide at room temperature. After stirring for 12 hours, the solvents were removed under reduced pressure, the residue suspended in 200 mL of 10% citric acid and extracted 3X 500 mL of diethyl ether. The combined ether extract were washed with 500 mL of brine, dried ($MgSO_4$) and concentrated to dryness. The residue was dissolved in 250 mL of toluene, heated to reflux for 12 hours, then concentrated to dryness under reduced pressure. Purification by medium pressure chromatography over silica gel, eluting with 15%

ethyl acetate/hexanes gave 3.2 g of the 3R-lactone as a clear foam. Further elution with the same solvents gave 6.15 g of the 3S-lactone as a white solid.

Step I: Preparation of N′-(1,1-dimethylethoxycarbonyl)-5(S)-amino-4(S)-(1′,1′-dimethylethyl-1,1-dimethylsilyloxy)-6-phenyl-2(R)-(phenylmethyl)-hexanoic acid

(3R,5S,1′S)-3-Benzyl-5-(1-((1,1-dimethylethoxycarbonyl)amino)-2-phenylethyl)dihydrofuran-2-(3H)-one, 0.5g, was dissolved in 30 mL of a 2:1 mixture of ethylene glycol dimethyl ether/water, and to it was added 5 mL of 1M lithium hydroxide at room temperature. After stirring for 1 hour, the solvent was removed in vacuo and the residue partitioned between 20 mL chloroform and 20 mL 10% citric acid. The layers were separated and the aqueous phase extracted with 3 X 20 mL chloroform. The combined organic layers were dried ($Na_2SO_4$) and the solvent removed to yield 0.46 g of the crude hydroxy acid. This residue was dissolved in 5 mL of dry DMF and 0.845 g tert-butyl dimethylsilyl chloride and 0.725 g of imidazole were added. After stirring for 18 hours, the reaction was poured into 50 mL of water and extracted with 3 X 20 mL of ethyl acetate. The combined organic extracts were washed with 3 X 20 mL of 10% citric acid, 1 X 20 mL of water, 3 X 10 mL of saturated aqueous solution of $Na_2CO_3$, and 20 mL of brine. After drying ($Na_2SO_4$), the solvent was removed and the resulting residue dissolved in a mixture of 5 mL of THF, 5 mL of glacial acetic acid, and 2 mL of water. The mixture was stirred for 4 hours, then poured into 50 mL of water and extracted with 3 X 20 mL of ether. The combined ether extracts were washed with 2 X 20 mL of water, brine, dried ($Na_2SO_4$), and the solvent removed. Purification by medium pressure chromatography over silica gel, eluting with $MeOH/CHCl_3$ gave 0.53 g of the product as a white solid.

Step J: Resolution of 1-amino-2-hydroxyindane.

The known racemate of the title compound was resolved via the diastereomeric phenylalanine amides, according to the method of Rittle, et. al. (Tetrahedron Letters 28,521 (1987). Saponification of the less polar amide gave 1(S)-amino-2(R)hydroxyindane ($\alpha_D$ = -58°, c=1.0, chloroform).

Step K: Preparation of N-2(R)-hydroxy-1(S)-indanyl-5(S)-(1,1-dimethylethoxycarbonyl)amino-4(S)(1′-1′-dimethylethyl-1,1-dimethylsilyloxy)-6-phenyl-2(R)-(phenylmethyl)hexanamide

A sample of 5(S)-(1,1-dimethylethoxycarbonyl amino-4(S)-(1′,1′-dimethylethyl-1,1-dimethylsilyloxy)6-phenyl-2(R)-(phenylmethyl)hexanoic acid (700 mg, 1.33 mmol) prepared as described in Step 1, was dissolved in 2.5 mL of dry DMF. HOBT (197 mg, 1.46 mmol) and EDC (280 mg, 1.46 mmol) were added. The mixture was stirred for 5 minutes and 200 mg of 1(S)-amino-2(R)-hydroxyindane (1.34 mmol) was added followed by 0.41 mL of triethylamine (2.9 mmol). After 24 hours the product was partitioned between ethyl acetate and 10% citric acid. The organic phase was washed with brine and dried ($Na_2SO_4$). The solvent was evaporated and the residue was chromatographed on silica gel (2% methanol/chloroform). There was obtained from this process 0.86 g of the desired product as an oil (98%).

Step L: Preparation of N-2(R)-hydroxy-1(S)-indanyl-5(S)-(1,1-dimethylethoxycarbonyl)amino-4(S)hydroxy-6-phenyl-2(R)-(phenylmethyl)hexanamide

The product of Step K was dissolved in 5 mL of IM tetrabutylammonium fluoride. After 48 hours the reaction mixture was poured into 10% citric acid and the precipitated product was isolated by filtration. The solid was dissolved in a mixture of THF and ethyl acetate, washed with water and brine, and evaporated to yield the 0.68 g of the title compound.

Step M: Preparation of N-2(R)-hydroxy-1(S)-indanyl-5(S)-amino-4(S)-hydroxy-6-phenyl-2(R)-(phenyl methyl)hexanamide

The product of Step L was dissolved in a mixture of 200 mL methylene chloride and 50 mL trifluoroacetic acid. After 30 minutes the solvents were evaporated and the residue was dissolved in ethyl acetate. This solution was washed with 10% $NaHCO_3$ and brine, dried and evaporated. The solid residue was triturated with ether to give 0.42 g of the desired compound as a white solid.

Step N: N-(2(R)-hydroxy-1(S)-indanyl)-5(S)-(3,6,9,12-tetraoxatridecyloxycarbonylamino)-4(S)-hydroxy-2(R)-phenylmethyl-6-phenylhexanamide

To a suspension of N-(2(R)-hydroxy-1(S)-indanyl)-5(S)-amino-4(S)-hydroxy-6-phenyl-2(R)-(phenylmethyl)-hexanoic amide (1.147 g, 2.58 mmol) in methylene chloride was added 0.36 mL triethylamine (2.6 mmol) and 946 mg (2.7 mmol) of N-1,3,6,9,12,15-hexoxa-2-oxo-hexadecyl-succinimide. The mixture was stirred overnight, diluted with methylene chloride and the solution washed sequentially with 10% citric acid, 5% $NH_4OH$ and brine. The solution was dried and evaporated and the residue was chromatographed on silica gel eluting with 1-2% MeOH in chloroform. The product (0.95 g) was obtained as a solid by trituration with ether, m.p. 148-150°C. Analysis.

Calc. for $C_{38}H_{50}N_2O_9$:
C, 67.24; H, 7.42; N, 4.13.
Found: C, 67.07; H, 7.23; N, 4.07.

EXAMPLE 2

N-(2(R)-hydroxy-1(S)-indanyl)-5(S)-(3,6,9,12-tetraoxatridecyloxycarbonylamino)-4(S)-hydroxy-2(R)-(3-phenylprop-2-en-1-yl)-6-phenylhexanamide

Step A: Preparation of N-2(R)-hydroxy-1(S)-indanyl)-5(S)-amino-4(S)-hydroxy-6-phenyl-2(R)-(3-phenylprop-2-en-1-yl)hexanamide

The title compound was prepared by the methods described in Example 1, Steps B-M, substituting cinnamyl bromide for the benzyl bromide employed in Step G.

Step B: Tetraethylene glycolmonomethyl ether (146 mg, 0.70 mmol) was dissolved in 2 mL THF with 121 mg p-nitrophenyl chloroformate and 0.84 mL (0.60 mmol) triethylamine was added. After 30 minutes 100 mg (0.21 mmol) of the product of Step A dissolved in 2 mL 1:1 THF/DMF and 0.54 mL triethylamine were added. After stirring 48 hours 100 mL water was added. After 24 hours the mixture was partitioned between ethyl acetate and 5% ammonium hydroxide. The organic layer was washed with water and brine and dried. Evaporation of the solvent gave a yellow solid which was chromatographed on silica with methanol/ chloroform.
The title compound (58 mg) was obtained pure by ether trituration. Analysis Calc. for $C_{40}H_{52}N_2O_9$:

C, 68.16; H, 7.44; N, 3.97.
Found: C, 68.09; H, 7.76; N, 4.03.

EXAMPLES 3-8

By the methods described in example 1 and example 2 and employing the appropriate alcohol in place of tetraethylene glycolmonomethyl ether, also were prepared:

N-(2(R)-hydroxy-1(S)-indanyl)-5(S)-(3,6,9,-trioxadecycloxycarbonylamino)-4(S)-hydroxy-2(R)-phenylmethyl-5-phenylhexanamide. Analysis Calc. for $C_{36}H_{46}N_2O_8$:

C, 68.12; H, 7.30; N, 4.41.
Found: C, 68.17; H, 7.26; N, 4.39.

N-(2(R)-hydroxy-1(S)-indanyl)-5(S)-(3,6,-dioxaheptylox ycarbonylamino)-4(S)-hydroxy-2(R)-phenylmethyl-6-phenylhexanamide. Analysis Calc. for $C_{34}H_{42}N_2O_7$:

C, 69.13; H, 7.17; N, 4.74.
Found: C, 69.03; H, 7.07; N, 4.77.

N-(2(R)-hydroxy-1(S)-indanyl)-5(S)-(3-oxabutyloxycarbonylamino)-4(S)-hydroxy-2(R)-phenylmethyl-6-phenylhexanamide. Analysis Calc. for $C_{36}H_{38}N_2O_6$:

C, 70.31; H, 7.01; N, 5.12.
Found: C, 70.42; H, 6.86; N, 5.08.

N-(2(R)-hydroxy-1(S)-indanyl)-5(S)-(3,6,9-trioxadecycloxycarbonylamino)-4(S)-hydroxy-2(R)-(3-phenylprop-2-enyl)-6-phenylhexanamide. Analysis Calc. for $C_{38}H_{48}N_2O_8$:

C, 69.07; H, 7.32; N, 4.24.
Found: C, 69.23; H, 7.66; N, 4.35.

N-(2(R)-hydroxy-1(S)-indanyl)-5(S)-(3,6,-dioxaheptyloxycarbonylamino)-4(S)-hydroxy-2(R)-(3-phenylprop-2-en-1-yl)-6-phenylhexanamide. Analysis Calc. for $C_{36}H_{44}N_2O_7$:

C, 70.11; H, 7.19; N, 4.54.
Found: C, 70.02; H, 7.24; N, 4.58.

N-(2(R)-hydroxy-1(S)-indanyl)-5(S)-(3-oxabutyloxycarbonylamino)-4(S)-hydroxy-2(R)-(3-phenylprop-2-en-1-yl)-6-phenylhexanamide. Analysis Calc. for $C_{34}H_{40}N_2O_6$:

C, 71.31; H, 7.04; N, 4.89.
Found: C, 71.54; H, 7.14; N, 4.94.

## EXAMPLE 9

Preparation of N-(2-benzimidazolymethyl)-5(S)-(3, 6, 9-trioxadecyloxycarbonylamino)-4(S)-hydroxy-6-phenyl-2(R)-(3-phenylprop-2-en-1-yl)hexanoyl isoleucyl amide

Step A: Preparation of N-(1,1-dimethylethoxycarbonyl)isoleucyl(succinimide

A 15 g (0.065 mole) quantity of Boc-isoleucine, 8.2 g of N-hydroxysuccinimide, and 13.7 g of EDC were dissolved in 80 mL of DMF. After stirring at 25°C for 18 hours, the solution was added to 500 mL of water and extracted with 3 x 200 mL of ethyl acetate. The organic layers were combined, washed with 4 x 200 mL portions of water, 200 mL of brine, and dried ($Na_2SO_4$). Filtration and concentration in vacuo gave 20.2 g (95%) of Step A product, mp 62-64°C.

Step B: Preparation of N-(2-benzimidazolylmethyl-N'-(1,1-dimethylethoxycarbonyl)isoleucyl amide

The product of Step A, 4.6 g (14 mmol), was dissolved in 70 mL of 1,2-dimethoxyethane, and to it were added 5.9 g (28 mmol) of 2-aminomethyl-benzimidazole dihydrochloride and 7.8 mL (56 mmol) of triethylamine. After stirring at 25°C for 18 hours, the solvent was removed in vacuo and the residue was dissolved in 250 mL of ethyl acetate. This solution was washed with 3 x 100 mL portions of water, 100 mL of brine, and dried ($Na_2SO_4$). Filtration and concentration in vacuo, gave 4.4 g (87%) of Step B product, after chromatography on silica gel ($CH_2Cl_2$:$CH_3OH$, 95:5).

Step C: Preparation of N-(2-benzimidazolylmethyl)isoleucyl amide

The product of Step B, 2 g (5.6 mmol), was dissolved in 50 mL of ethyl acetate and cooled to -25°C. Hydrogen chloride gas bubbled into the solution for 0.75 hour or until TLC indicated complete reaction. Nitrogen was bubbled into the reaction as it was left to warm to ambient temperature . Concentration in vacuo gave 1.6 (86%) of Step C product.

Step D: Preparation of N-(2-benzimidazolymethyl)-5(S)-(1,1-dimethylethoxycarbonylamino)-4(S)-hydroxy-6-phenyl-2(R)-(3-phenylprop-2-en-1-yl)hexanoyl isoleucyl amide

The 5(S)-(1,1-dimethylethoxycarbonylamino)-4(S)-hydroxy-6-phenyl-2(R)-(3-phenylprop-2-en-1-yl) hexanoic acid as an intermediate in Example 2 was coupled to the product of Step C using the conditions of Example 1, Step K.

Step E: Preparation of N-(2-benzimidazolymethyl)-5(S)-(3,6,9-trioxadecyloxycarbonylamino)-4(S) hydroxy-6-phenyl-2(R)-(3-phenylprop-2-en-1-yl)hexanoyl isoleucyl amide

The product of Step D was converted by the method of Example 1, Step M and Example 2, Step B to the title compound, mp 164-166 Anal. Calcd for $C_{43}H_{57}H_5O_8 \cdot 0.5\ H_2O$: C, 66:13; H, 7.49; N, 8:97 Found C, 66.12; H, 7.44; N, 8.90.

EXAMPLE 10

Preparation of N-(2(R)-hydroxy-1(S)-indanyl)-5(S)-[(2-hydroxyethoxycarbonyl)amino]-4(S)-hydroxy-6-phenyl-2-(R)-(phenylmethyl)]-hexanamide

Step A: Preparation of 2-(1′,1′-dimethylethyl-1,1-diphenylsilyloxy)ethoxy p-nitrophenyl carbonate

To a solution of 2′(1′,1′-dimethylethyl-1,1-diphenylsilyloxy)ethanol (544 mg) and N-methylmorpholine (202 mg) in 30 mL methylene chloride was added 403 mg p-nitrophenyl chloroformate. After 18 hours at room temperature, the mixture was shaken with 10% citric acid and the organic layer was dried and evaporated. The resulting oil was treated with hexane to precipitate p-nitrophenol and the filtrate was evaporated to give the desired product containing 30% unreacted alcohol. This was used without purification in the next step.

Step B: Preparation of N-(2(R)-hydroxy-1(S)-indanyl)-5(S)-[2-((1′,1′-dimethylethyl-1,1-diphenylsilyloxy)ethoxycarbonyl)amino]-4(S)-hydroxy-6-phenyl-2-(R)-(phenylmethyl)]-hexanamide

To a solution of 95 mg of N-(2(R)-hydroxy-1(S)-indanyl)-5(S)-[(1,1-dimethylethoxycarbonyl)amino]-4(S)-hydroxy-6-phenyl-2-(R)-(phenylmethyl)]hexanamide in 6 ml of methylene chloride at 0°C was added 2 ml of trifluoroacetic acid. After 3 hours, the reaction was evaporated to dryness and the crude residue dissolved in 3 ml of DMF and 0.2 ml of triethylamine. A solution of 150 mg of the compound form step A in 2 ml of DMF was added and the reaction stirred at room temperature for 18 hours after which time it was concentrated to dryness and purified (2 mm $SiO_2$ prep plate, 5% MeOH/$CHCl_3$) to afford the product.

Step C: Preparation of N-(2(R)-hydroxy-1(S)-indanyl)-5(S)-[(2-hydroxyethoxycarbonyl)amino]-4(S)-hydroxy-6-phenyl-2-(R)-(phenylmethyl)]-hexanamide

The product from step B above was dissolved in 4 ml of THF and 1 ml of a 1M solution of tetrabutylammonium fluoride was added. After 2 hours at room temperature, the reaction was diluted with 25 ml of water, extracted with ethyl acetate followed by methylene chloride and the organics were combined, dried, filtered and concentrated to a yellow solid. The residue was chromatographed (2 mm $SiO_2$ prep plate, 5% MeOH/$CHCl_3$) to afford the product. m.p. 181-183°C.

EXAMPLE 11

The preparation of N'-[(2-hydroxyethoxy)carbonyl-5(S)-amino-4(S)-hydroxy-6-phenyl-2(R)-(3-phenylprop-3en-1-yl)hexanoyl]-N-isoleucine(2-benzimidazolylmethyl)amide

In the same manner as described in example 10, 5(S)-amino-4(S)-hydroxy-6-phenyl-2(R)-(3-phenylprop-2-en-1-yl)hexanoyl]-N-isoleucine (2-benzimidazolymethyl)amide (prepared in Example 9) was reacted with [2-(1',1'-dimethylethyl-1,1-diphenylsilyloxy)ethoxy]-p-nitrophenoxycarbamate to yield N'-5(S)-[2-((1',1'-dimethylethyl-1,1-diphenylsilyloxy)-ethoxycarbonyl)amino]-4(S)-hydroxy-6-phenyl-2(R)-(3-phenylprop-2-en-1-yl) hexanoyl]-N-isoleucine(2-benzimidazolylmethyl)amide. This compound was desilylated as described in Example 9 Step C to yield N'-[((2-hydroxyethoxy)carbonyl-5(S)-amino-4(S)-hydroxy-6-phenyl-2(R)-(3-phenylprop-2-en-1-yl)hexanoyl]-N-isoleucine(2-benzimidazolymethyl)amide. m.p. 202-204°C.

EXAMPLE 12

Preparation of N-[(cis)-2(R)-hydroxy-1(S)-indanyl)-5(S)-[(2-hydroxyethoxycarbonyl)amino]-4(S)-hydroxy-6-phenyl-2-(R)-(3-phenylprop-2-en-1-yl)hexanamide

Step A: Preparation of 2-(t-butyldimethylsilyloxy)ethylsuccinimid-1-yl carbonate

2-(t-Butyldimethylsilyloxy)ethanol (17.3 g) was dissolved in 200 mL methylene chloride, cooled to -40°C and 9.9 g triphosgene was added. A solution of 8.0 mL pyridine in 50 mL methylene chloride was added dropwise. The cooling bath was removed and the mixture was stirred for 2 hours at room temperature. After cooling to -5°C a solution of 12.4 g N-hydroxysuccinimide and 15 mL triethylamine in 120 mL acetonitrile was added dropwise. The cooling bath was removed, the mixture was allowed to warm to room temperature and concentrated in vacuo. The residue was partitioned between ethyl acetate and 10% citric acid. The organic phase was dried and evaporated and the residue was chromatographed on silica gel to give the title compounds as a white solid. $^1$H NMR ($CDCl_3$): δ 4.39 (2H, t), 3.90 (2H, t), 2.82 (4H, s), 0.90 (9H, s), 0.10 (6H, s).

Step B: Preparation of N-(2(R)-hydroxy-1(S)-indanyl)-5(S)-[(2-hydroxyethoxycarbonyl)amino]-4(S)-hydroxy-6-phenyl-2-(R)-(3-phenylprop-2-en-1-yl)hexanamide

The product of Step A (270 mg) was combined with N-(2(R)-hydroxy-1(S)-indanyl)-5(S)-amino-4(S)-hydroxy-6-phenyl-2-(R)-(3-phenylprop-2-en-1-yl)hexanamide (400 mg, prepared in Example 2) in methylene chloride and 120 μL triethylamine was added. After stirring overnight the solution was washed with 10% citric acid, dried and evaporated. The crude product was desilylated as described in Example 1, Step L to give the title compound, mp; 218.5-219.2°C. Anal. Calcd for $C_{33}H_{38}N_2O_6$:

C, 70.93; H, 6.86; N, 5.02.
Found: C, 71.09; H, 7.00; N, 4.99.

EXAMPLE 13

Preparation of N-(2(R)-hydroxy-1(S)-indanyl)-5(S)-(3,6,9,12-tetraoxatridecyloxycarbonylamino)-4(S)-hydroxy-6-phenyl-2(R)-(4-hydroxyphenyl)methylhexaneamide

The known compound (see EPO 0,337,714 A2) N-(2(R)-hydroxy-1(S)-indanyl)-5(S)-(1,1-dimethylethoxycarbonylamino)-4(S)-hydroxy-6-phenyl-2(R)-(4-hydroxyphenyl)methylhexaneamide was converted to the title compound using the methods described in Examples 1 and 2. Anal. Calcd for $C_{38}H_{50}N_2O_{10}\cdot 0.32H_2O$:

C, 64.95; H, 7.30; N, 3.99.
Found: C, 64.96; H, 7.22; N, 3.99.

EXAMPLE 14

Preparation of N-[2-(R)-hydroxy-1(S)-indanyl]-5(S)-[(3-hydroxy)propoxycarbonyl]amino-4(S)-hydroxy-6-phenyl-2(R)-(3-phenylpropyl)hexanamide

Step A: Preparation of 1-[3-(benzyloxy)propoxycarbonyloxy]pyrrolidin-2,5-dione

To a stirred solution of phosgene in toluene (10 ml of 12.5% commercial solution) at 0°C, was added 1.05 g of (6.3 mmol) 3-benzyloxy-1-propanol. The resulting solution was stirred at 0 to 23°C for 24 hours. After this period, nitrogen was bubbled through for 15 minutes to remove excess of phosgene. The mixture was then evaporated to dryness under reduced pressure. The oily residue was dissolved in anhydrous acetonitrile (25 ml), cooled to 0°C and 950 mg (8 mmol) of N-hydroxy succinimide, followed by 1.5 ml of triethylamine were added to this solution. The resulting mixture was then stirred at 0°C to room temperature for 2 hours. The reaction was quenched with 10 ml of saturated sodium bicarbonate solution and extracted with ethyl acetate (2x). The combined extracts were dried over anhydrous sodium sulfate and concentrated in vacuo. The residue was chromatographed over silica gel with 50% ethyl acetate in hexane to afford 2.0 g of title carbonate as a colorless oil.

Step B: Preparation of N-[2(R)-hydroxy-1(S)-indanyl]-5(S)-[(3-hydroxy)propoxycarbonyl]amino-4(S)-hydroxy-6-phenyl-2(R)-[3-phenylpropanyl]hexanamide

1-[3-(Benzyloxy)propoxycarbonyloxy]pyrrolidin-2,5-dione 0.388 g, was dissolved in 5 mL of dry methylene chloride, 0.4 g of N'-(2(R)-hydroxy-1(S)-indanyl)-5(S)-amino-4(S)-hydroxy-6-phenyl-2(R)-(3-phenylprop-2-en-1-yl)methylhexanamide (obtained in Example 2) was added. Triethylamine was added to the stirring solution until the pH was 8.5. After stirring for 12 hours at room temperature, the reaction was poured into 5 mL of water and extracted with 3x15 mL of ethyl acetate. The combined organic extracts are washed with 10% citric acid, water, saturated aqeuous $NaHCO_3$ solution and dried over anhydrous $Na_2SO_4$. Evaporation of the solvent gave a residue which was chromatographed over silica gel (5% methanol/chloroform) to afford 0.49 g of carbamate derivative as a white solid. This product (.03 g) was dissolved in 3:1 ethyl acetate/methanol (5 ml) and the resulting solution was hydrogenated over 10% Pd-C (15 mg) under balloon pressure at room temperature for 12 hours. After this period, the solution was filtered and evaporated to give a residue which was chromatographed over silica gel (50% ethyl acetate/hexane) to furnish 0.02 g of the title compound as a white solid, mp 177°-180°C.

EXAMPLE 15

Preparation of N-[2(R)-hydroxy-1(S)-indanyl]-5(S)-[(3-methoxy)propoxycarbonyl]amino-4(S)-hydroxy-6-phenyl-2(R)-(3-phenylprop-2-en-1-yl]hexanamide

The title compound, mp 173°-175°C, was prepared using 3-methoxypropan-1-ol, and N-(2(R)hydroxy-1(S)-indanyl)-5(S)-amino-4(S)-hydroxy-6-phenyl-2(R)-(3-phenylprop-2-en-1-yl)-hexanamide prepared in Example 2, in the method of Example 1, Steps A and N.

EXAMPLE 16

N-(2(R)-hydroxy-1(S)-indanyl)-5(S)-(13,13-dimethyl-3,6,9,12-tetraoxatetradecyloxycarbonylamino)-4(S)-hydroxy-2(R)-phenylmethyl-6-phenylhexanamide

Step A: Preparation of 13,13-dimethyl-3,6,9,12-tetraoxatetradecanol

Tetraethylene glycol (10 g) was dissolved in 85 mL t-butanol and 4.5 mL c sulfuric acid was added dropwise with stirring. After 3 days a solution of 7.2 g NaOH in 150 mL methanol, was added, the suspension was slurried with Celite and filtered. The filtrate was evaporated and the solid residue was extracted with ether. The ether solution was dried and evaporated. Three materials were detected by TLC (10% $MeOH/CHCl_3$ $R_f$: 0.33, starting material; 0.48, title compound; 0.67, bis-t-butyl ether of starting material). The desired component was isolated by silica gel chromatography with 1-2% $MeOH/CHCl_3$.

Step B: Preparation of N-(2(R)-hydroxy-1(S)-indanyl)-5(S)-(13,13-dimethyl-3,6,9,12-tetraoxatetradecyloxycarbonylamino)-4(S)-hydroxy-2(R)-phenylmethyl-6-phenylhexanamide

The product of Step A was converted to the succinimidyl carbonate as described in Example 12, Step A. This reagent was used as described in Example 1, Step N to prepare the title compound, mp 150.3-151.5. Anal. Calcd for $C_{41}H_{56}N_2O_9$:

| | |
|---|---|
| | C, 68.31; H, 7.83; N, 3.89. |
| Found: | C, 68.30; H, 7.99; N, 3.90. |

EXAMPLE 17

Assay for Inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30°C for 1 hour. Various concentrations of inhibitor in 1.0 ul DMAO were added to 25 ul of the peptide solution in water. The reaction is initiated by the addition of 15 ul of 0.33 nM protease (0.11 ng) in a solution of 0.133 M Na acetate pH 5.5 and 0.26% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. Compound A showed an $IC_{50}$ value about 5nM.

While the foregoing specification teaches the principles of the present invention, with examples provided for the purpose of illustration, it will be understood that the practice of the invention encompasses all of the usual variations, adaptations, or modifications, as come within the scope of the following claims and its equivalents.

**Claims**

1. A compound of the formula:

A-G-B-B-J I,

wherein A is

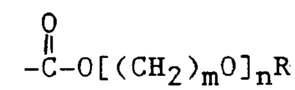

where n = 1-6, m may be 1-3 within each repeating unit n, and R is either hydrogen or $C_{1-4}$ alkyl.

G is

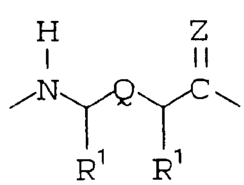
,

wherein Z is O, S, or NH, and

$R^1$ is independently

1) hydrogen
2)

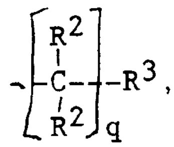

3) -OR, wherein R is H, or $C_{1-4}$ alkyl,
4) $-NR_2$,
5) $C_{1-4}$ alkylene-$R^3$; wherein q is 0-5 and $R^2$ is independently
a) hydrogen,
b) hydroxy, or
c) $C_{1-4}$-alkyl;

$R^3$ is
a) hydrogen,
b) aryl, unsubstituted or substituted with one or more of
i) halo,
ii) hydroxy,
iii) $-NH_2$, $-NO_2$, -NHR, or $-NR_2$, wherein R is H, or $C_{1-4}$ alkyl,
iv) $C_{1-4}$ alkyl,
v) $C_{1-3}$ alkoxy,
vi) -COOR,
vii)

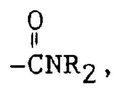

viii) $-CH_2NR_2$,
ix)

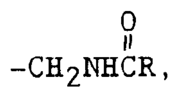

x) CN,
xi) $CF_3$,
xii)

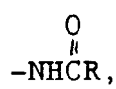

xiii) aryl $C_{1-3}$ alkoxy,
xiv) aryl,
xv) $-NRSO_2R$,
xvi) $-OP(O)(OR_x)_2$ wherein $R_x$ is H or aryl, benzyl or a metal ion,
xvii)

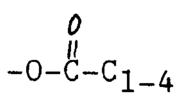

alkyl substituted with one or more of amine or quaternary amine;

$R^3$ may also be
c) 5 or 6 membered heterocycle including up to 3 heteroatoms selected from N, O, and S, any of which heterocycle may be unsubstituted or substituted with one or more of

i) halo,
ii) hydroxy,
iii) $-NH_2$, $-NHR$, $-NR_2$,
iv) $C_{1-4}$ alkyl,
v) $C_{1-3}$ alkoxy,
vi) -COOR,
vii)

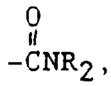

viii) $-CH_2NR_2$,
ix)

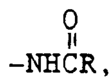

x) -CN,
xi) $CF_3$,
xii) $-NHSO_2R$,
xiii) $-OP(O)(OR_x)_2$ wherein $R_x$ is H or aryl, or
xiv)

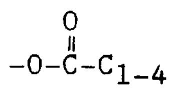

alkyl substituted with one or more of amine or quaternary amine;
xv) SR, S(O)R and $S(O_2)R$;

d) $C_{1-6}$ alkyl or $C_{1-6}$ alkenyl, unsubstituted or substituted with one or more of
i) hydroxy,
ii) $C_{1-4}$ alkyl,
iii) $-NH_2$, $-NHR$, $-NR_2$,
iv)

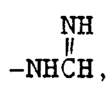

v)

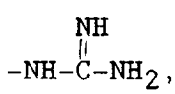

vi) -COOH,
vii)

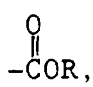

viii) -SR,S(O)R and $S(O)_2R$,
ix) $-SO_2NHR$,
x) $C_{1-4}$ alkyl sulfonyl amino or aryl sulfonyl amino,

xi) -CONHR,
xii)

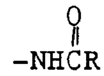,

xiii) -OR,
xiv) aryl $C_{1-3}$ alkoxy or,
xv) aryl;

e) $C_{3-7}$ cycloalkyl unsubstituted or substituted with one or more of

i) hydroxy,
ii) $C_{1-4}$ alkyl,
iii) $-NH_2$, -NHR, $-NHR_2$,
iv)

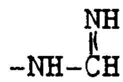,

v)

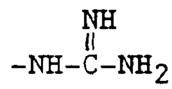,

vi) -COOH,
vii)

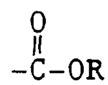,

viii) -SR,S(O)R and $S(O_2)R$
ix) $-SO_2NH_2$,
x) alkyl sulfonylamino or aryl sulfonylamino,
xi) -CONHR, or
xii)

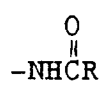;

f) a 5- to 7-membered carbocyclic or 7- to 10-membered bicyclic carbocyclic ring which is either saturated or unsaturated, the carbocyclic ring being unsubstituted or substituted with one or more of

i) halo
ii) -OR, wherein R is H or $C_{1-4}$ alkyl,
iii)

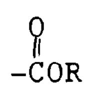,

iv)

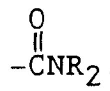

v) $-CH_2NR_2$,
vi) $-SO_2NR_2$ or $-S(O)_y$ R wherein y is 0,1 or 2,
vii) $-NR_2$,
viii)

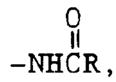

ix) $C_{1-4}$ alkyl,
x) phenyl,
xi) $-CF_3$, or
xii)

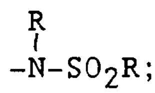

$R^9$ is -OH or $-NHR^{10}$, wherein $R^{10}$ is -H,

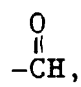

$-C_{1-4}$- alkyl or -COOR; and

Q is

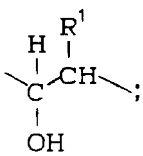 ; 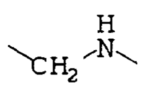 ; 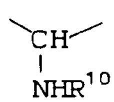 ; 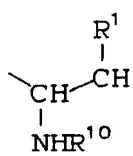 ; 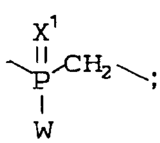 ; or 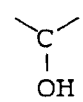 ,

wherein $R^1$ and $R^{10}$ are defined above; $X^1$ is O, S, or NH; and

W is
1) OH,
2) $NH_2$,
3) OR, or
4) NHR;

B is, independently, absent, or

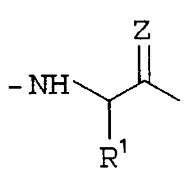 ,

J is

1) $YR^{11}$ wherein:

Y is O or NH, and

$R^{11}$ is

a) H;

b) $C_{1-6}$ alkyl, unsubstituted or substituted with one or more of

i) $-NR^6_2$,

ii) -OR,

iii) $-NHSO_2C_{1-4}$ alkyl,

iv) $-NHSO_2$ aryl, or $-NHSO_2$ (dialkylaminoaryl),

v) $-CH_2OR$,

vi) $C_{1-4}$ alkyl,

vii)

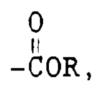

viii)

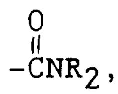

ix)

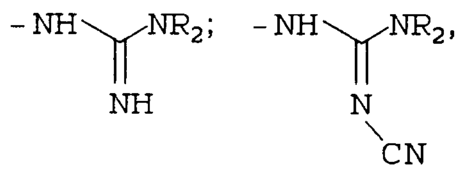

x)

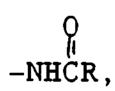

xi)

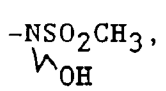

xii)

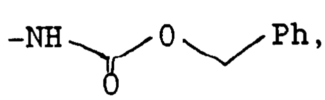

xiii) $-NR_3^{\oplus}A^{\ominus}$ wherein $A^{\ominus}$ is a counterion,

xiv) $-NR^{12}R^{13}$ wherein $R^{12}$ and $R^{13}$ are the same or different and are $C_{1-5}$ alkyl joined together directly to form a 5-7 membered heterocycle,

xv) aryl,

xvi) -CHO,
xvii) $-OP(O)(OR_x)_2$ wherein $R_x$ is H or aryl, or
xviii)

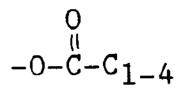

alkyl substituted with one or more of amine or quaterany amine;
c) $-(CH_2CH_2O)_nCH_3$ or $-(CH_2CH_2O)_nH$;
2) $-N(R^{11})_2$,
3) $-NR^{12}R^{13}$ wherein $R^{12}$ and $R^{13}$ are defined above, or
4)

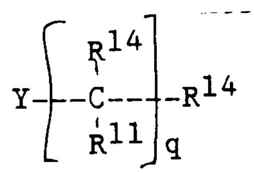

wherein:

Y, $R^{11}$, and q are defined above, and
$R^{14}$ is
a) hydrogen;
b) aryl unsubstituted or substituted with one or more of
i) halo,
ii) -OR, wherein R is H or $C_{1-4}$ alkyl,
iii)

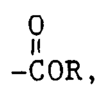

iv)

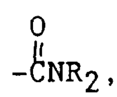

v) $-CH_2NR_2$,
vi) $-SO_2NR_2$,
vii) $-NR^2$,
viii)

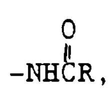

ix) $C_{1-4}$ alkyl,
x) phenyl,
xi) $-CF_3$,
xii)

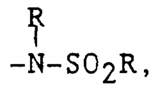

xiii) $-C_{1-4}$ alkyl-$NR_2$,

xiv) $-OP(O)(OR_x)_2$ wherein $R_x$ is H or aryl, or

xv)

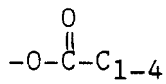

alkyl substituted with one or more of amine or quaternary amine;

c) heterocycle, unsubstituted or substituted with one or more of

i) halo,

ii) -OR, wherein R is H, $C_{1-4}$ alkyl, or $C_{1-4}$ alkenyl,

iii)

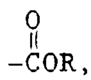

iv)

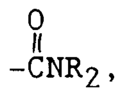

v) $-CH_2NR_2$,

vi) $-SO_2NR_2$,

vii) $-NR_2$,

viii)

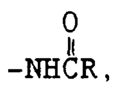

ix) $C_{1-4}$ alkyl,

x) phenyl,

xi) $-CF_3$,

xii)

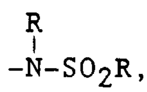

xiii) phenyl $C_{1-4}$ alkyl,

xiv)

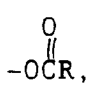

xv) $-OP(O)(OR_x)_2$ wherein $R_x$ is H or aryl, or

xvi)

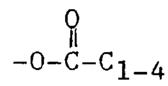

alkyl substituted with one or more of amine or quaternary amine;

d) A 5- to 7-membered carbocyclic or 7- to 10-membered bicyclic carbocyclic ring which is either saturated or unsaturated, the carbocyclic ring being unsubstituted or substituted with one or more of

i) halo,

ii) -OR, wherein R is H or $C_{1-4}$ alkyl,

iii)

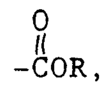

iv)

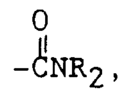

v) $-CH_2NR_2$,

vi) $-SO_2NR_2$,

vii) $-NR_2$,

viii)

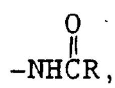

ix) $C_{1-4}$ alkyl,

x) phenyl,

xi) $-CF_3$;

xii)

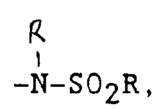

xiii) $-OP(O)(OR_x)_2$ wherein $R_x$ is H or aryl, or

xiv)

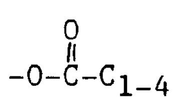

alkyl substituted with one or more of amine or quaternary amine;

or pharmaceutically acceptable salts, hydrates or esters thereof.

**2.** A compound according to Claim 1, wherein:

A is

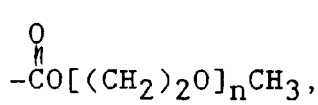

n=1-4; and

G is

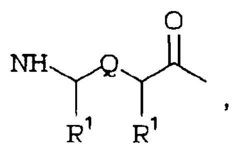

3. A compound according to Claim 2, wherein:

A is

$$-\overset{O}{\overset{\|}{C}}O[(CH_2)_2O]_nCH_3,$$

n=1-4;

G is

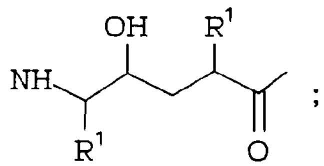

and

B is absent or present once.

4. A compound according to Claim 3, wherein:

A is

$$-\overset{O}{\overset{\|}{C}}O[(CH_2)_2O]_nCH_3,$$

n=1-4;

G is

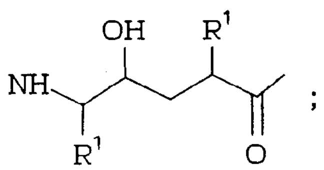

B is absent;

J is $NHR^{14}$ and $R^{14}$ is a substituted 5- to 7-membered carbocyclic or heterocyclic ring or a substituted 7- to 10-membered bicyclic carbocycle or heterocycle which may be saturated or unsaturated.

5. A compound according to Claim 4 wherein

J is indan, substituted once or twice with OH.

6. The compound,

N-(2(R)-hydroxy-1(S)-indanyl-5(S)-(3,6,-9,12-tetraoxatridecyloxycarbonylamino)-4(S)-hydroxy-2(R)-phenylmethyl-6-phenyl-hexanamide,

N-(2(R)-hydroxy-1(S)-indanyl-5(S)-(3,6,-9,12-tetraoxatridecyloxycarbonylamino)-4(S)-hydroxy-2(R)-phenylprop-2-en-1-yl)-6-hexanamide,
N-(2(R)-hydroxy-1(S)-indanyl)-5(S)-(3,6,9-trioxadecyloxycarbonylamino)-4(S)-hydroxy-2(R)-phenylmethyl-6-phenylhexanamide,
N-(2(R)-hydroxy-1(S)-indanyl)-5(S)-(3,6-dioxaheptyloxycarbonylamino)-4(S)-hydroxy-2(R)-phenylmethyl-6-phenylhexanamide,
N-(2(R)-hydroxy-1(S)-indanyl)-5(S)-(3-oxabutyloxycarbonylamino)-4(S)-hydroxy-2(R)-phenylmethyl-6-phenylhexanamide,
N-(2(R)-hydroxy-1(S)-indanyl)-5(S)-(3,6,9-trioxadecyloxycarbonylamino)-4(S)-hydroxy-2(R)-(3-phenylprop-2-en-1-yl)-6-phenylhexanamide,
N-(2(R)-hydroxy-1(S)-indanyl)-5(S)-(3,6-dioxaheptyloxycarbonylamino)-4(S)-hydroxy-2(R)-(3-phenylprop-2-en-1-yl)-6-phenylhexanamide,
N-(2(R)-hydroxy-1(S)-indanyl)-5(S)-(3-oxabutyloxycarbonylamino)-4(S)-hydroxy-2(R)-(3-phenylprop-2-en-1-yl)-6-phenylhexanamide,
N-(2-benzimidazolylmethyl)-5(S)-(3,6,9-trioxadecyloxycarbonylamino)-4(S)-hydroxy-6-phenyl-2(R)-(3-phenylprop-2-en-1-yl)hexanoyl isoleucylamide,
N-[(cis)-2(R)-hydroxy-1(S)-indanyl)-5(S)-[(2-hydroxyethoxycarbonyl)amino]-4(S)-hydroxy-6-phenyl-2(R)(phenylmethyl)]hexanamide,
N'-[2-hydroxyethoxy)carbonyl-5(S)-amino-4(S)-hydroxy-6-phenyl-2(R)-(3-phenylprop-2-en-1-yl)hexanoyl]-N-isoleucine-(2-benzimidazolylmethyl)amide,
N-[(cis)-2(R)-hydroxy-1(S)-indanyl]-5(S)-[(2-hydroxyethoxycarbonyl)amino)-4(S)-hydroxy-6-phenyl-2(R)-(3-phenylprop-2-en-1-yl)hexanamide,
N-(2(R)-hydroxy-1(S)-indanyl)-5(S)-(3,6,9,12-tetraoxatridecyloxycarbonylamino)-4(S)-hydroxy-6-phenyl-2(R)-(4-hydroxyphenyl)methylhexanamide,
N-(2(R)-hydroxy-1(S)-indanyl)-5(S)-[(3-hydroxy)propoxycarbonyl]amino-4(S)-hydroxy-6-phenyl-2(R)-[3-phenylpropanyl]hexanamide,
N-(2(R)-hydroxy-1(S)-indanyl)-5(S)-[(3-methoxy)propoxycarbonyl]amino-4(S)-hydroxy-6-phenyl-2(R)-[3-phenylprop-2-en-1-yl]hexanamide, or
N-(2(R)-hydroxy-1(S)-indanyl)-5(S)-(13,13-dimethyl-3,6,9,12-tetraoxatetradecyloxycarbonylamino)-4(S)-hydroxy-2(R)-phenylmethyl-6-phenylhexanamide,
or pharmaceutically acceptable salt or ester therof.

7. The compound
N-(2(R)-hydroxy-1(S)-indanyl)-5(S)-(3,6,9,12-tetraoxatriadecyloxycarbonylamino)-4(S)-hydroxy-2(R)-phenylmethyl-6-phenylhexanamide,
or pharmaceutically acceptable salt or ester therof.

8. A pharmaceutical composition comprising a compound as in any of Claims 1-7 and a pharmaceutically acceptable carrier.

9. The pharmaceutical composition of Claim 8 for use in the treatment of AIDS, in the prevention of infection by HIV, in the treatment of infection of HIV, or in the inhibition of HIV protease.

10. The use of a compound as claimed in claim 1 for the manufacture of a medicament for the treatment of AIDS.

11. The use of a compound as claimed in claim 1 for the manufacture of a medicament for the prevention or treatment of infection by HIV.

12. The use of a compound as claimed in claim 1 for the manufacture of a medicament for the inhibition of HIV protease.